# EUROPEAN PATENT APPLICATION

(11) **EP 2 270 134 A1**
(43) Date of publication of application: **05.01.2011**
(21) Application number: 09164541.6
(22) Date of filing: 03.07.2009
(51) Int. Cl.: C12N 1/20, C12N 1/36, A23L 1/30, C12R 1/01

(54) **Oxidative stress resistant Bifidobacteria**

(71) Applicant: Nestec S.A., 1800 Vevey (CH)
(72) Inventor: Mozzetti, Valeria, 8005, ZURICH (CH); Lacroix, Christophe, 8802, KILCHBERG (CH); Grattepanche, Franck, 8051, ZURICH (CH); Arigoni, Fabrizio, 1204, GENEVE (CH); Rezzonico, Enea, 1066, EPALINGES (CH); Berger, Bernard, 1610, CHATILLENS (CH)
(74) Representative: Marquardt, Ulf

(57) **Abstract**

The present invention relates in general to the field of *Bifidobacteria.* In particular the present invention relates to the field of *Bifidobacteria* with an improved resistance to oxidative stress. The inventors have identified a Bifidobacterium that exhibits for example an improved H₂O₂ - resistance.

## Description

The present invention relates in general to the field of *Bifidobacteria.* In particular the present invention relates to the field of *Bifidobacteria* with an improved resistance to H₂O₂.

Probiotic bacteria are selected for their health-promoting properties. As probiotics exert their benefit as living organism (Guarner, F. and G. J. Schaafsma. 1998. "Probiotics". Int.J.Food Microbiol. 39:237-238), the bacteria should survive the gastro-intestinal track conditions. In industrial applications, large-scale production requires the additional ability of the probiotics to tolerate food processing and storage. These production steps are characterized by different stresses compromising the good survival of the bacteria. Amongst them, high or low temperature, high osmotic pressure, oxidation, and humidity are likely key factors.

Some species of probiotic bacteria are in particular known to have a poor temperature, oxygen, or spray-drying tolerance. Prominent examples of such probiotics with a poor stress tolerance are, e.g., *Bifidobacteria,* in particular *Bifidobacterium longum* (Simpson, P. J., C. Stanton, G. F. Fitzgerald, and R. P. Ross. 2005. "Intrinsic tolerance of Bifidobacterium species to heat and oxygen and survival following spray drying and storage". J.Appl.Microbiol. 99:493-501).

The anaerobic nature of bifidobacteria in particular, requires customized fermentation conditions to facilitate large-scale production and additional effort is usually needed to preserve cell viability during processing and storage.

Extreme differences in oxygen tolerance occur throughout the bacterial kingdom. Aerobic bacteria have evolved elaborate defence systems to protect themselves against the damage caused by reactive oxygen species (ROS). However even for these bacteria, oxygen can be toxic should these defence systems become impaired. Mainly three different reactive oxygen species (ROS) may be formed, depending whether oxygen accepts one, two or three electrons to form (i) superoxide radicals (O²⁻), (ii) hydrogen peroxide (H₂O₂) or (iii) hydroxyl radicals (OH), respectively (Imlay, J. A., 2002, Advances in Microbial Physiology, Vol 46 46:111-153.). H₂O₂ causes DNA damage and oxidation of proteins. Bacteria may use several enzymes to eliminate this ROS, including catalases, NADH peroxidases and reductases.

To address this problem and to increase the survival rate of probiotics during food production, several approaches are presently used, including microencapsulation, addition of protective agents, oxygen-impermeable packaging, and improvement of the growth or processing conditions.

However, these approaches are complex and laborious to carry out and often require the addition of further compounds that may have side effects and that may be unwanted in the final food-product.

Consequently, it was the object of the present invention to overcome the problems of the prior art and to provide the art with probiotic *Bifidobacteria,* that exhibit an improved resistance against oxidative stress and/or an improved oxygen tolerance and with a method to increase the number of viable probiotics in a composition.

This object of the present invention is solved by a *Bifidobacterium* in accordance with claim 1, by a composition in accordance with claim 2 and by a use in accordance with claim 8.

The present inventors have applied natural selection methods and have searched for *Bifidobacteria* strains with an increased resistance towards reactive oxygen species, such as for example H₂O₂.

One typical probiotic bacterium is *Bifidobacterium longum.*

The present inventors were able to identify and isolate *Bifidobacterium longum* NCC2916 which allowed achieving the object of the present invention. *Bifidobacterium longum* NCC2916 - a derivative of *Bifidobacterium longum* NCC2705 obtained by natural selection - surprisingly exhibits an improved oxygen tolerance compared with Bifidobacteria of the prior art. Strains obtained by natural selection have the advantage that they solve the object of the present invention without having been modified by means of gene technology.

In particular, B. longum strain NCC2916 is showing an improved resistance to H₂O₂.

Consequently, one embodiment of the present invention is *Bifidobacterium longum* NCC2916.

*Bifidobacterium longum* NCC2916 has been deposited under the Budapest treaty with the CNCM; Institut Pasteur, 25; Rue du Docteur Roux; F-75724 Paris Cedex 15 as CNCM I-4053.

*Bifidobacterium longum* NCC2705 has been deposited under the Budapest treaty with the CNCM; Institut Pasteur, 25; Rue du Docteur Roux; F-75724 Paris Cedex 15 as CNCM I-2618.

The present invention also relates to a composition comprising *Bifidobacterium longum* NCC2916.

For the purpose of the present invention a composition is considered as containing *Bifidobacterium longum* NCC2916 if it contains *Bifidobacterium longum* NCC2916 cells - living or non-replicative, any bacterial fraction of *Bifidobacterium longum* NCC2916, any fraction of a culture comprising *Bifidobacterium longum* NCC2916, and/or the culture media or a part thereof that was used to cultivate *Bifidobacterium longum* NCC2916.

Probiotic *Bifidobacteria,* when administered, confer a health benefit on the host. The health benefits of *Bifidobacteria* are manifold and include aid in digestion, association with a lower incidence of allergies, prevention of some forms of tumour growth and weight management. The composition and/or the *Bifidobacterium longum* NCC2916 of the present invention may consequently be used to confer these health benefits to a subject.

The composition of the present invention may be any composition but is preferably a food product, an animal food product or a pharmaceutical composition. For example, the composition may be a nutritional composition, a nutraceutical, a drink, a food additive or a medicament.

Preferably, composition is intended for humans, pets or livestock, while pets or livestock may be selected from the group consisting of dogs, cats, guinea pigs, rabbits, pigs, cattle, sheep, goats, horses and/or poultry.

The composition may comprise at least one carbohydrate source, at least one lipid source and/or at least one protein source.

As protein source any suitable dietary protein may be used, for example animal proteins (such as milk proteins, meat proteins and egg proteins); vegetable proteins (such as soy protein, wheat protein, rice protein, and pea protein); mixtures of free amino acids; or combinations thereof. Milk proteins such as casein and whey, and soy proteins are particularly preferred. The proteins may be intact or hydrolysed or a mixture of intact and hydrolysed proteins. It may be desirable to supply partially hydrolysed proteins (degree of hydrolysis between 2 and 20%), for example for animals believed to be at risk of developing cows' milk allergy. If hydrolysed proteins are required, the hydrolysis process may be carried out as desired and as is known in the art. For example, a whey protein hydrolysate may be prepared by enzymatically hydrolysing the whey fraction in one or more steps. If the whey fraction used as the starting material is substantially lactose free, it is found that the protein suffers much less lysine blockage during the hydrolysis process. This enables the extent of lysine blockage to be reduced from about 15% by weight of total lysine to less than about 10% by weight of lysine; for example about 7% by weight of lysine which greatly improves the nutritional quality of the protein source.

If the composition includes a fat source, the fat source preferably provides 5% to 40% of the energy of the composition; for example 20% to 30% of the energy. A suitable fat profile may be obtained using a blend of canola oil, corn oil and high-oleic acid sunflower oil.

The source of carbohydrates preferably provides 40% to 80% of the energy of the composition. Any suitable carbohydrate may be used, for example sucrose, lactose, glucose, fructose, corn syrup solids, maltodextrins, and mixtures thereof.

Dietary fibre may also be added if desired. Dietary fibre passes through the small intestine undigested by enzymes and functions as a natural bulking agent and laxative. Dietary fibre may be soluble or insoluble and in general a blend of the two types is preferred. Suitable sources of dietary fibre include soy, pea, oat, pectin, guar gum, gum Arabic, fructooligosaccharides, galacto-oligosaccharides, sialyl-lactose and oligosaccharides derived from animal milks. A preferred fibre blend is a mixture of inulin with shorter chain fructooligosaccharides. Preferably, if fibre is present, the fibre content is between 2 and 40 g/l of the composition as consumed, more preferably between 4 and 10 g/l.

The composition may also contain minerals and micronutrients such as trace elements and vitamins in accordance with the recommendations of Government bodies such as the USRDA.

For example, the composition may contain per daily dose one or more of the following micronutrients calcium, magnesium, phosphorus, iron, zinc, copper, iodine, selenium, Vitamin C, Vitamin B1, Vitamin B6, Vitamin B2, niacin, Vitamin B12, folic acid, biotin, Vitamin D, Vitamin E.

One or more food grade emulsifiers may be incorporated into the composition if desired; for example diacetyl tartaric acid esters of mono- and di-glycerides, lecithin and mono- and di-glycerides. Similarly suitable salts and stabilisers may be included.

The composition may be intended for oral, parenteral or topical administration. Oral application includes for the purpose of the present invention enteral administration.

The composition may comprise at least one other kind of other food grade bacteria. "Food grade bacteria" means bacteria that are used and generally regarded as safe for use in food. The food grade bacteria may be selected from the group consisting of lactic acid bacteria, and/or bifidobacteria. Preferred are probiotic bacteria "Probiotic bacteria" mean microbial cell preparations or components of microbial cells with a beneficial effect on the health or well-being of the host. (Salminen S, Ouwehand A. Benno Y. et al "Probiotics: how should they be defined" Trends Food Sci. Technol. 1999:10 107-10).

The composition may further contain at least one prebiotic. "Prebiotic" means food substances intended to promote the growth of probiotic bacteria in the intestines. Prebiotics are for example defined by Glenn R. Gibson and Marcel B. Roberfroid, Dietary Modulation of the Human Colonic Microbiota: Introducing the Concept of Prebiotics, J. Nutr. 1995 125: 1401-1412.The prebiotic may be selected from the group consisting of oligosaccharides and optionally contains fructose, galactose, mannose, soy and/or inulin; and/or dietary fibers.

The composition of the present invention may further contain protective hydrocolloids (such as gums, proteins, modified starches), binders, film forming agents, encapsulating agents/materials, wall/shell materials, matrix compounds, coatings, emulsifiers, surface active agents, solubilizing agents (oils, fats, waxes, lecithins etc.), adsorbents, carriers, fillers, co-compounds, dispersing agents, wetting agents, processing aids (solvents), flowing agents, taste masking agents, weighting agents, jellifying agents, gel forming agents, antioxidants and antimicrobials. The composition may also contain conventional pharmaceutical additives and adjuvants, excipients and diluents, including, but not limited to, water, gelatine of any origin, vegetable gums, ligninsulfonate, talc, sugars, starch, gum arabic, vegetable oils, polyalkylene glycols, flavouring agents, preservatives, stabilizers, emulsifying agents, buffers, lubricants, colorants, wetting agents, fillers, and the like. In all cases, such further components will be selected having regard to their suitability for the intended recipient.

The composition of the present invention may contain any number of *Bifidobacteria* in accordance with the present invention. In principle, a single *Bifidobacterium* that arrives in the intestine and is able to generate a colony is sufficient to generate a beneficial effect on the host.

It is clear to those skilled in the art that an ideal dose will depend on the subject to be treated, its health condition, sex, age, or weight, for example. The dose to be ideally used will consequently vary but can be determined easily by those of skill in the art.

However, generally, it is preferred if the composition of the present invention comprises between 10³ and 10¹⁰ cfu and/or between 10³ and 10¹⁰ cells of *Bifidobacterium longum* NCC2916 per daily dose. It may also comprise between 10³ and 10¹² cfu and/or between 10³ and 10¹² cells of *Bifidobacterium longum* NCC2916 per g of the dry weight of the composition.

The present invention also relates to the use of a *Bifidobacterium longum* NCC2916 to increase the number of living probiotic bacteria, for example *Bifidobacterium longum,* in a product. Similarly, the present invention relates to the use of the composition of the present invention to increase the number of living probiotic bacteria, for example *Bifidobacterium longum,* in a product. The uses of the present invention serve also to increase the number of probiotic bacteria, in particular of *Bifidobacteria,* in a product after exposure to stress for the probiotic bacteria.

Stress may be an exposure to oxidative species, for example in a concentration of 200 ppm, for example for 30 seconds to 2 hours.

The oxidative species are not particularly limited. Very common reactive oxygen species include for example •O²⁻, the superoxide anion; H₂O₂, hydrogen peroxide; •OH, the hydroxyl radical; ROOH, organic hydroperoxide; RO•, alkoxy radicals; ROO•, peroxy radicals; HOCI, hypochlorous acid; OONO-, peroxynitrite; and/or NO•. Preferably, the reactive oxygen species are selected from the group consisting of H₂O₂, •O²⁻, •OH˙, and combinations thereof

Those skilled in the art will understand that they can freely combine all features of the present invention described herein, without departing from the scope of the invention as disclosed. In particular, features described for the use of the present invention may be applied to the composition of the present invention and vice versa.

Further advantages and features of the present invention are apparent from the following Examples and Figures.

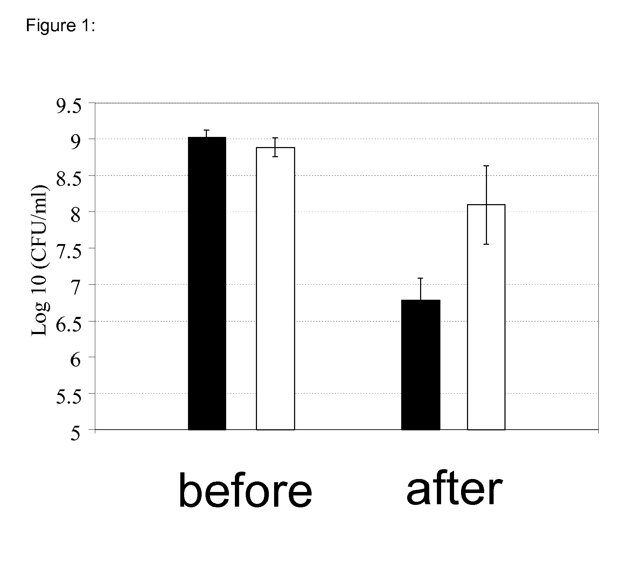

Figure 1 shows viable cells counts of 16 h overnight cultures after 4 h in 200 ppm H₂O₂. NCC2916 (white bars) and wild type NCC2705 (black bars). p-values refer to a t-test with the wild type

### Example 1:

### Identification and isolation of NCC2916

Approximately 10¹¹ cfu of NCC2705 were resuspended in 10 ml 40 ppm H₂O₂ for 80 minutes. A 1 ml volume of cell suspension was subsequently plated on MRS agar. A colony visible within 48 h was sub-cultured 2 times overnight (16 h) in MRS broth and frozen at -80°C for further analyses.

To compare H₂O₂ resistance of wild type cells and the new variant NCC2916 , frozen cell samples (16 h overnight cultures) from wild-type and variants cultures at the same cell densities were thawed and aliquots of 1 ml were centrifuged (6'000 g, 2 min at room temperature). The pellet was resuspended in 10 ml 200 ppm H₂O₂ solution. After 4 h the cell suspension was diluted in buffer and plated. Colony counts were measured after 48 h. The test was performed in triplicate (Fig 1). H₂O₂ tolerance of NCC2916 was stable for at least 70 generations.

## Claims

1. *Bifidobacterium longum* NCC2916.

2. Composition comprising a *Bifidobacterium* in accordance with claim 1.

3. Composition in accordance with claim 2 **characterized in that** the composition is a food product, an animal food product or a pharmaceutical composition.

4. Composition in accordance with one of claims 2-3 **characterized in that** it further contains at least one prebiotic.

5. Composition in accordance with claim 4 **characterized in that** the prebiotic is selected from the group consisting of oligosaccharides and optionally contains fructose, galactose, mannose, soy and/or inulin; and/or dietary fibers.

6. Composition in accordance with one of claims 1-5 **characterized in that** it comprises between 10³ and 10¹⁰ cfu and/or between 10³ and 10¹⁰ cells of *Bifidobacterium* NCC2916 per daily dose.

7. Composition in accordance with one of claims 1-5 **characterized in that** it comprises between 10³ and 10¹² cfu and/or between 10³ and 10¹² cells of *Bifidobacterium* NCC2916 per g of the dry weight of the food composition.

8. Use of a *Bifidobacterium* in accordance with claims 1 or of a composition in accordance with claims 1-7 to increase the number of living probiotic bacteria in a product.

9. Use in accordance with claim 8 to increase the number of probiotic bacteria in a product after exposure to stress for the probiotic bacteria.

10. Use in accordance with claim 9 wherein the stress is an exposure to oxidative species, in a concentration of 200 ppm for 30 seconds to 2 hours.

11. Use in accordance with claim 10 wherein the oxidative species is selected from the group consisting of H₂O₂, •O²⁻, •OH, and combinations thereof.
